# EUROPEAN PATENT SPECIFICATION

(11) **EP 1 838 248 B1**
(45) Date of publication and mention of the grant of the patent: **06.03.2013**
(21) Application number: 06718593.4
(22) Date of filing: 17.01.2006
(51) Int. Cl.: A61F 2/28, A61F 2/44, A61L 27/36, A61F 2/30, A61F 2/00

(54) **EXPANDABLE OSTEOIMPLANT**
AUSDEHNBARES KNOCHENIMPLANTAT
IMPLANT OSSEUX EXPANSIBLE

(30) Priority: 14.01.2005 US 643901 P
(43) Date of publication of application: 03.10.2007
(73) Proprietor: Warsaw Orthopedic, Inc., Warsaw, IN 46581 (US)
(72) Inventor: MORRIS, John, W., Beachwood, New Jersey 08722 (US); RICHARDS, Cristy, J., Matawan, New Jersey 07747 (US); SHIMP, Lawrence, A., Morganville, New Jersey 07751 (US); MILLER, Timothy, R., Holmdel, New Jersey 08722 (US); ROSENTHAL, Daniel, Short Hills, New Jersey 07078 (US)
(74) Representative: O'Connell, Maura
(86) International application number: PCT/US2006/001540
(87) International publication number: WO 2006/076712

(56) References cited:
- WO-A-00/35510
- WO-A-02/47587
- WO-A-98/17209
- WO-A-2004/108023
- US-A- 6 030 635
- US-A1- 2002 120 338

## Description

### FIELD OF THE INVENTION

This invention relates generally to an osteoimplant, and more specifically to an osteoimplant comprising an expandable, biocompatible material. The expandable material may include demineralized cancellous chips, demineralized cortical fibers, expandable polymers, other suitable materials, or combinations of these. The osteoimplant has a first state and an expanded state, and it may be used with another device or on its own. The description also discloses generally a method for manufacturing the osteoimplant.

### BACKGROUND OF THE INVENTION

Surgical procedures for fusing adjacent vertebrae to treat various pathologies are well known. Implants for such procedures take a wide variety of shapes, forms, and materials, from bone to titanium inert materials, rigid and elastic, circular cylindrical, wedge shapes, and cages with or without openings to accept bone fusion promoting material. The implants are dimensioned and shaped to fill a predetermined disc space between the adjacent vertebra to be fused.

During spinal fusion surgery, intimate contact between the vertebral endplates and fusion-promoting devices and materials is beneficial for a timely, successful outcome. With current techniques, the surgeon often implants an intervertebral body fusion device (bone or non-bone) to maintain restored intervertebral height, provide stability to the motion segment, transfer load from one vertebral body to the next, and allow for a fusion mass to develop between the two vertebral bodies. Prior to inserting the device, the vertebral endplates are prepared to create a uniform endplate surface, increasing the likelihood of endplate-device contact and subsequent fusion mass formation. Depending on the case, indications, concavity of the endplate, and skill of the surgeon, however, uniform endplate preparation may be difficult to achieve. To assist in the creation of a fusion mass, the surgeon will often pack the implant with material that is osteoconductive. Alternatively, or in addition to the osteoconductive material, the surgeon may pack the implant with material that is osteoinductive. Once implanted, however, the osteoconductive and/or osteoinductive material in the intervertebral space is not assured of being in close contact with both vertebral endplates.

Bone grafts are commonly used in treating bone fractures and defects. Further, bone grafts may be used as part of spinal surgery to encourage bone fusion with or through the implant Bone grafting procedures are directed to a diverse array of medical interventions for complications such as fractures involving bone loss, injuries, or other conditions necessitating immobilization by fusion (such as for the spine or joints), and other bone defects that may be present due to trauma, infection, or disease. Bone grafting involves the surgical transplantation of pieces of bone within the body, and generally is effectuated through the use of graft material acquired from a human source.

Orthopedic autografts or autogenous grafts involve source bone acquired from the individual receiving the transplantation. Thus, this type of transplant moves bony material from one location in a body to another location in the same body, and has the advantage of producing minimal immunological complications. However, it is not always possible or even desirable to use an autograft.

In use, the grafts may be placed, for example, in a host bone and serve as the substructure for supporting new bone tissue growth from the host bone. The grafts are sculpted to assume a shape that is appropriate for insertion at the fracture or defect area, and often require fixation to that area as by screws or pins.

WO00/35510 to Osteotech refers to implantable, biocompatible, osteogenic bone grafts which comprise at least one zone of impermeability to soft tissue ingrowth which is integral with the bone graft, and it further refers to application of the bone graft to a bone repair site leading to selective rapid new bone ingrowth and inhibition or prevention of soft tissue, e.g. gingival, epithelial, connective and/or muscle tissue, ingrowth in those areas adjacent to the zone(s) of impermeability to soft tissue ingrowth.

WO98/17209 to SDGI Holdings, Inc., refers to spinal spacers provided for fusion of a motion segment. Said spacers include a load bearing member having a wall sized for engagement within a spce between adjacent vertebrae to maintain the space and an effective amount of an osteogenic composition to stimulate osteoinduction.

US2002/0120338 to Boyer et al., refers to selectively partially and demineralised bone derived implants.

### SUMMARY OF THE INVENTION

An osteoimplant and a method for manufacturing the osteoimplant are disclosed. More specifically, an osteoimplant is provided according to claim 1, comprising demineralized bone particles having a first state and an expanded state. The osteoimplant may be used with another device or on its own.

In the first state, the osteoimplant may be inserted into a device such as an intervertebral body fusion device. Thus, the osteoimplant may alternatively be referred to as a bone insert. The osteoimplant may be rehydrated to expand to an increased size, for example as far as permitted by the confines of the intervertebral body fusion device and spinal endplates, thereby aiding in greater vertebral endplate contact and conformity in spinal surgery.

Alternatively, the osteoimplant may be used on its own, for example in a surgical opening or anatomical void, where it is desirable that the osteoimplant expand from a first state to an expanded state.

This application refers to various patents, patent applications, journal articles, and other publications. The following documents are referred to: U.S. Patent No. 6,294,187 to Boyce et al. for LOAD-BEARING OSTEOIMPLANT, METHOD OF ITS MANUFACTURE AND METHOD OF REPAIRING BONE USING SAME; U.S. Patent No. 6,123,731 to Boyce et al. for OSTEOIMPLANT AND METHOD FOR ITS MANUFACTURE; U.S. Patent No. 6,706,067 to Shimp et al. for SPINAL INTERVERTEBRAL DEVICE AND METHOD OF MAKING; Currant Protocols in Molecular Biology, Current Protocols in Immunology, Current Protocols in Protein Science, and Current Protocols in Cell Biology, John Wiley & Sons, N.Y., edition as of July 2002; Sambrook, Russell, and Sambrook, Molecular Cloning: A Laboratory Manual, 3rd ed., Cold Spring Harbor Laboratory Press, Cold Spring Harbor, 2001; Rodd 1989 "Chemistry of Carbon Compounds," Volumes 1-5 and Supplements, Elsevier Science Publishers, 1989; "Organic Reactions," Volumes 1-40, John Wiley and Sons, New York, NY, 1991; March 2001, "Advanced Organic Chemistry," 5th ed. John Wiley and Sons, New York, NY. In the event of a conflict between the specification and any of the references, the specification shall control. Where numerical values herein are expressed as a range, endpoints are included.

While multiple embodiments are disclosed, still other embodiments of the invention will be apparent to those skilled in the art from the following detailed description, which shows and describes illustrative embodiments of the invention. As will be realized, the invention is capable of modifications in various obvious aspects, all without departing from the scope of the invention. Accordingly, the drawings and detailed description are to be regarded as illustrative in nature and not as restrictive.

### BRIEF DESCRIPTION OF THE DRAWINGS

Figure 1 illustrates an osteoimplant in accordance with one embodiment of the present invention.

Figure 2 illustrates an intervertebral fusion device for receipt of an osteoimplant in accordance with one embodiment of the present invention.

Figure 3a illustrates one embodiment of the osteoimplant of the present invention that has been inserted into the intervertebral fusion device of Figure 2 and is in a first state.

Figure 3b illustrates one embodiment of the osteoimplant of the present invention that has been inserted into the intervertebral fusion device of Figure 2 and is in an expanded state.

Figure 4 illustrates an intervertebral device spaced between substantially uniform vertebral endplates.

Figure 5 illustrates an intervertebral device spaced between non-uniform vertebral endplates.

Figure 6 illustrates the intervertebral device of Figure 5 after expansion.

Figure 7 illustrates an intervertebral device with the osteoimplant in accordance with one embodiment of the present invention and illustrates the expansion of the osteoimplant and the bone growth present at the site at approximately 4 weeks.

Figure 8 illustrates an osteoimplant after 0 minutes rehydration.

Figure 9 illustrates an osteoimplant after 30 minutes rehydration.

Figure 10 illustrates Insertion/Expansion testing of an osteoimplant in accordance with one embodiment of the present invention.

Figure 11 illustrates Insertion/Expansion testing of an osteoimplant in accordance with one embodiment of the present invention.

Figure 12 illustrates Insertion/Expansion testing of an osteoimplant in accordance with one embodiment of the present invention.

Figure 13 illustrates sample expansion of an osteoimplant in a vertebral cage in accordance with one embodiment of the present invention.

### DEFINITIONS

The term "osteoimplant" herein is utilized in its broadest sense and is not intended to be limited to any particular material, shapes, sizes, configurations, or applications. It may include any material, such as allograft, xenograft, or synthetic material, used to promote or support bone healing.

The term "shape" or "shaped" as applied to the osteoimplant herein refers to a determined or regular form or configuration, in contrast to an indeterminate or vague form or configuration (as in the case of a lump or other solid mass of no special form), and is characteristic of such materials as sheets, plates, disks, cones, pins, screws, tubes, teeth, bones, portion of bone, wedges, cylinders, threaded cylinders, and the like.

The term "osteogenic" as used herein refers to the ability of a substance to induce new bone formation via the participation of living cells from within the substance. Stated otherwise, the term osteogenic shall be understood as referring to the ability of the osteoimplant to enhance or accelerate the ingrowth of new bone tissue by one or more mechanisms such as osteogenesis, osteoconduction, and/or osteoinduction.

The term "osteoconductive" as used herein refers to the ability of a substance or material to provide biologically inert surfaces that are receptive to the growth of new host bone. The term "osteoinductive" shall be understood to refer to the ability of a substance to recruit cells from the host that have the potential for repairing bone tissue.

The term "osteoconforming^{™}" as used herein refers to the ability of a substance conform to biological geometries in vivo.

Use of the expression "bone-derived elements" refers to pieces of bone in any variety of sizes, thicknesses, and configurations, including particles, fibers, strips, thin to thick sheets, etc., which can be obtained by milling, slicing, cutting, or machining whole bone.

The expression "bone particles" refers to pieces of bone and may comprise cancellous bone pieces, cortical bone pieces, or a combination thereof. The bone particles may be partially or fully demineralized and/or combined with nondemineralized bone particles and/or other materials. The bone particles may be obtained from cortical, cancellous, and/or corticocancellous bone that may be of autogenous, allogenic, and/or xenogenic origin. The bone particles may be powdered bone particles possessing a wide range of particle sizes ranging from relatively fine powders to coarse grains and even larger chips. The bone particles may range in size from approximately 1 mm to approximately 15 mm. The pieces of bone may alternatively be elongate, possessing relatively high median length to median thickness ratios may be utilized herein. Such elongate particles may be referred to as fibers. The elongate bone particles may have a median length of from about 2 to about 200 mm, a median thickness of from about 0.05 to about 2 mm, and a median width of from about 1 mm to about 20 mm.

The term "incorporation" as utilized herein refers to the biological mechanism whereby host tissue gradually removes portions of the osteoimplant of the invention and replaces those removed portions with native host bone tissue while maintaining strength. This phenomenon also is known in the scientific literature as "creeping substitution." The term "incorporation" utilized herein shall be understood as embracing what is known by those skilled in the art as "creeping substitution."

"Demineralized" refers to removal of minerals from bone. Demineralization may range from substantially complete (in which case the bone-derived elements are primarily collagen) to partial or superficial (in which case only the surfaces of the bone-derived elements present exposed collagen). Partial or superficial demineralization produces bone-derived elements having a surface binding region, namely, exposed collagen, while retaining a strengthening region, namely, the unaffected nondemineralized region of the bone-derived elements. The expression "surface-exposed collagen" refers to the result obtained by demineralizing bone-derived elements.

"Biocompatible material" refers to a material not having toxic or injurious effects on biological function that would be inappropriate in a specific application.

"Bioactive agent" or "bioactive compound" refers to a compound or entity that alters, inhibits, activates, or otherwise affects biological or chemical events. For example, bioactive agents may include, but are not limited to, osteogenic or chondrogenic proteins or peptides, anti-AIDS substances, anti-cancer substances, antibiotics, immunosuppressants, anti-viral substances, enzyme inhibitors, hormones, neurotoxins, opioids, hypnotics, anti-histamines, lubricants, tranquilizers, anti-convulsants, muscle relaxants and anti-Parkinson substances, anti-spasmodics and muscle contractants including channel blockers, miotics and anti-cholinergics, anti-glaucoma compounds, anti-parasite and/or anti-protozoal compounds, modulators of cell-extracellular matrix interactions including cell growth inhibitors and antiadhesion molecules, vasodilating agents, inhibitors of DNA, RNA or protein synthesis, antihypertensives, analgesics, anti-pyretics, steroidal and non-steroidal anti-inflammatory agents, anti-angiogenic factors, angiogenic factors, anti-secretory factors, anticoagulants and/or antithrombotic agents, local anesthetics, ophthalmics, prostaglandins, antidepressants, anti-psychotic substances, anti-emetics, imaging agents, and any other useful substance. In certain embodiments, the bioactive agent is a drug. In some embodiments, the bioactive agent is a growth factor, cytokine, extracellular matrix molecule or a fragment or derivative thereof, for example, a cell attachment sequence such as RGD. A more complete listing of bioactive agents and specific drugs suitable for use in the present invention may be found in "Pharmaceutical Substances: Syntheses, Patents, Applications," by Axel Kleemann and Jurgen Engel, Thieme Medical Publishing, 1999; the "Merck Index: An Encyclopedia of Chemicals, Drugs, and Biologicals," Edited by Susan Budavari et al., CRC Press, 1996; and the United States Pharmacopeia-25/National Formulary-20, published by the United States Pharmcopeial Convention, Inc., Rockville MD, 2001.

### DETAILED DESCRIPTION OF PREFERRED EMBODIMENTS

**INTRODUCTION**

An osteoimplant and a method for manufacturing the osteoimplant are disclosed. More specifically, a shaped osteoimplant is provided according to claim 1, comprising an expandable material having a first state and an expanded state that may be used with another device or on its own. A method for manufacturing such implant is disclosed. The expandable material may comprise demineralized bone particles such as demineralized cancellous chips, demineralized cortical fibers, a synthetic material such as a polymer, any other suitable material, or combinations thereof. The osteoimplant may alternatively be referred to as a bone insert.

**IMPLANT**

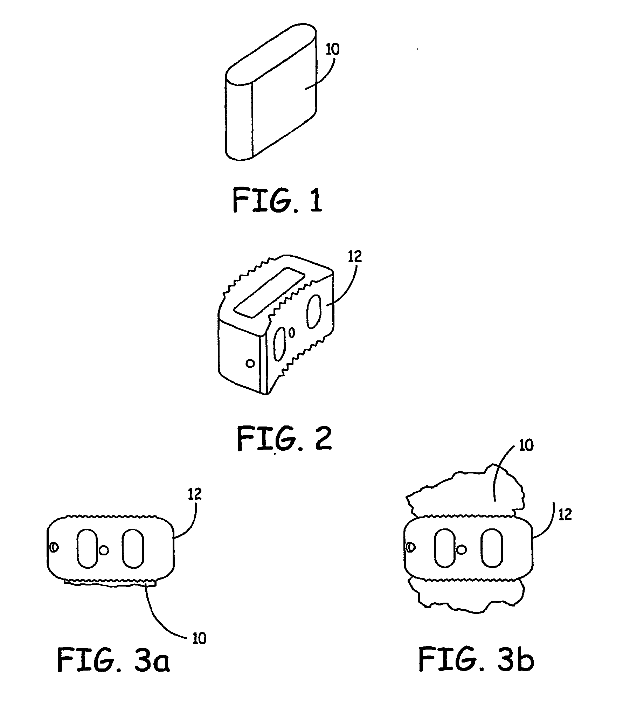

Figure 1 illustrates an osteoimplant 10 in accordance with one embodiment. The osteoimplant 10 is a shaped implant designed for expansion in vivo. Thus, when used in the context of vertebral fusion, the osteoimplant expands to the contours of the vertebral endplates, creating a matrix for cellular penetration to promote bone healing.

Generally, the osteoimplant comprises an expandable material. The expandable material may be natural or synthetic. The expandable material may comprise bone particles, a polymer, a hydrogel, a sponge, collagen, or other material. In one embodiment, the osteoimplant comprises bone allograft comprising demineralized bone particles. The demineralized bone particles comprise a blend of cortical and cancellous bone. For example, the osteoimplant may comprise demineralized cortical fibers and demineralized cancellous chips. Inductive cortical fibers are demineralized to expose naturally occurring growth factors found in bone. Demineralized cancellous chips create a healthy matrix for the incorporation of new bone and add advanced expansion characteristics.

Different materials and/or bone configurations may be used in alternative embodiments of the invention. For example, it may be useful in certain applications to use cortical bone fibers, cortical bone particles, or any other geometry of cortical bone pieces, whether or not demineralized, along with cancellous bone fibers, cancellous bone particles, or any other geometry of cancellous bone pieces, whether or not demineralized, or any mixture of combination of these. In some embodiments, the osteoimplant may comprise a synthetic material.

In addition to bone particles, an expandable polymer, a collagen sponge, compressed and/or dried hydrogels, or other materials may be used. In addition to expansion properties, the material may exhibit osteoinductive and/or osteoconductive properties. For example, cancellous bone particles may exhibit osteoconductive properties while demineralized cortical bone particles may exhibit osteoinductive properties.

Expansion properties may be imparted to the osteoimplant in any suitable manner. For example, expansion properties may be affected by the materials used to form the osteoimplant and/or by the manner used to form the osteoimplant. The osteoimplant may be configured to expand at least 10 percent, or from approximately 10 percent to approximately 1000 percent.

As is known in the art, some materials exhibit more expansion than others. The material used to form the implant may be selected based on the amount of expansion desired. The material may include coiled fibers, or it may be compressed, spring-loaded, or otherwise configured for expansion.

Compressing the material during formation may lead to subsequent expansion. Generally, increased compression leads to increased expansion characteristics in the osteoimplant. Compressed materials and certain noncompressed materials may be constrained such that, absent the constraint, the material is free to expand. A constrained material is one that embodies energy, such as a bent, spring-loaded, or coiled material, or any other material that is artificially prevented from expanding or conforming to its natural configuration. The constraint may comprise a membrane that partially or wholly surrounds the material. Thus, for example, the membrane may be provided around the material such that removal of the membrane permits the material to expand from its first state. Alternatively, the membrane may be a protective barrier such that removal of the membrane exposes the material to conditions that may activate expansion. Such membrane may be configured, for example, as a layer that disintegrates in vivo. More specifically, the membrane may be a biocompatible, biodegradable material such as a polyester based on polylactide (PLA), polyglycolide (PGA), polycaprolactone (PCL), and their copolymers, or it may be any other suitable material. Alternatively, the membrane may be a protective material or restraining barrier that is removed prior to implantation of the osteoimplant.

Expansion may be activated in any suitable manner. For example, expansion may be activated by exposure to air, water, blood, heat, removal of a constraint, or otherwise. In one embodiment, the osteoimplant may be compressed and dried. Upon exposure to liquid in vivo, the osteoimplant expands. In another embodiment, the osteoimplant may be compressed and constrained by a membrane. Upon exposure to liquid in vivo, the membrane disintegrates, and the osteoimplant expands. In yet another embodiment, the osteoimplant may be constrained by a protective material or restraining barrier. Upon removal of the material or barrier, the material expands as a function of time. In yet another embodiment, the osteoimplant has a first state at approximatel (60 degrees F) and an expanded state at approximatel 15,6°C 36,7°C (98 degrees F) such that, upon implantation in vivo and exposure to body heat, the osteoimplant expands. In a further embodiment, the osteoimplant is vacuum-sealed upon formation and packaging and, when unsealed and exposed to air, expands.

The direction of expansion may be controlled or uncontrolled. For example, in one embodiment, the osteoimplant may be constrained in directions other than the desired direction of expansion. In another embodiment, during formation of the osteoimplant, the osteoimplant may be compressed only in the direction of desired expansion. In still other embodiments, the osteoimplant may expand in any or all desired directions.

The osteoimplant may be configured to be load-bearing, may be configured for insertion into a load-bearing implant, or may be used in a non-load-bearing capacity.

In some embodiments, the osteoimplant may be shaped. Such shaping may comprise compressing the material in a mold and drying under compression. Drying may be done via freeze-drying, solvent drying, or heat-drying. The resulting osteoimplant is expansive. For example, in a specific embodiment, the resulting osteoimplant may be configured for expansion of approximately 2 to 10 mm upon rehydration. In alternate embodiments, the osteoimplant may be left unshaped and be used to fill a space in another implant or in a bone defect.

Generally, the osteoimplant exhibits osteoconforming™ characteristics. Thus, the osteoimplant expands upon rehydration in-vivo to conform to, for example, adjacent vertebral bodies. This maximizes the surface area contact between the graft and the endplate for optimal interface and cellular exchange. The osteoimplant may further exhibit osteoinductive and osteoconductive characteristics. Materials used to manufacture the osteoimplant may be chosen with deference to these characteristics. For example, in an osteoimplant comprising demineralized bone particles, demineralized cortical fibers provide osteoinductive characteristics, while demineralized cancellous chips provide osteoconductive characteristics and enhanced expansion. Thus, the cortical fibers contribute to the bone formation process while the cancellous chips provide optimal scaffold for vascularization and bone formation, thereby providing a healthy matrix for the new bone growth.

Mixtures or combinations of one or more types of bone particles and/or other materials may be employed. For example, one or more types of demineralized bone particles, cancellous or cortical, or expandable material may be employed in combination with nondemineralized bone particles, i.e., bone particles that have not been subjected to a demineralization process, or other materials. The exact composition of the implant, for example, the extent of demineralization of bone particles and the ratio of demineralized bone particles, nondemineralized bone particles, synthetic materials, and other materials may vary depending on the desired use of the osteoimplant. For example, osteoimplants that are to be used as an intervertebral fusion device may be provided with enhanced strength. Alternatively, or in addition, the osteoinductivity of the osteoimplant and/or providing a coherency or binding effect may be of greater importance, in which case a greater fraction of fully demineralized bone particles may be used. In situations where the osteoimplant is used on its own, for example in a surgical opening or anatomical void, the strength of the osteoimplant may take on greater importance, and superficially or partially demineralized bone particles and/or nondemineralized bone particles may be used in the osteoimplant.

The osteoimplant may optionally include additional biocompatible component(s) such as wetting agents, biocompatible binders, fillers, fibers, plasticizers, biostatic/biocidal agents, surface active agents, bioactive agents, and the like. Carriers also may be used where appropriate. Thus, in addition to containing bone-derived elements, the osteoimplant may optionally possess one or more other components such as reinforcing particles, fibers, fillers, bone-growth inducing substances such as growth factors, adhesives, plasticizers, flexibilizing agents, hydration facilitating agents, biostatic/biocidal agents, analgesics, hemostats, substances imparting radiopacity such as nondemineralized bone chips, metallic meshes, structural pins and screws, and the like. Examples of reinforcing particles include nondemineralized cortical and cancellous bone, and partially demineralized cortical and cancellous bone in any form, including particles, sheets, and shaped bone particles, as well as graphite or pyrolytic carbon or any other suitable material. Examples of fillers include mineral material such as hydroxyapatite, tricalcium phosphate and other calcium salts, bone powder, fully nondemineralized and partially or fully demineralized cortical and cancellous bone in any form, including particles such as demineralized bone powder, sheets, and shaped bone particles, graphite or pyrolytic carbon, bioglass or other bioceramic or natural or synthetic polymers, e.g., bioabsorbable polymers such as polyglycolide, polylactide, glycolide-lactide copolymer, and the like, and nonbioabsorbable materials such as starches, polymethyl methacrylate, polytetrafluoroethylene, polyurethane, polyethylene, and nylon, other suitable materials, or any combination of these. Suitable plasticizers, flexibilizing agents and hydration facilitating agents include liquid polyhydroxy compounds such as glycerol, monacetin, diacetin, and mixtures thereof. Suitable biostatic/biocidal agents include antibiotics, povidone, sugars, and mixtures thereof; suitable surface agents include the biocompatible nonionic, cationic, anionic and amphoteric surfactants, and mixtures thereof. Suitable bioactive agents include any compound or entity that alters, inhibits, activates, or otherwise affects biological or chemical events. For example, bioactive agents may include, but are not limited to, osteogenic or chondrogenic proteins or peptides, anti-AIDS substances, anti-cancer substances, antibiotics, immunosuppressants, anti-viral substances, enzyme inhibitors, hormones, neurotoxins, opioids, hypnotics, anti-histamines, lubricants, tranquilizers, anti-convulsants, muscle relaxants and anti-Parkinson substances, anti-spasmodics and muscle contractants including channel blockers, miotics and anti-cholinergics, anti-glaucoma compounds, anti-parasite and/or anti-protozoal compounds, modulators of cell-extracellular matrix interactions including cell growth inhibitors and antiadhesion molecules, vasodilating agents, inhibitors of DNA, RNA or protein synthesis, antihypertensives, analgesics, anti-pyretics, steroidal and non-steroidal anti-inflammatory agents, anti-angiogenic factors, angiogenic factors, anti-secretory factors, anticoagulants and/or antithrombotic agents, local anesthetics, ophthalmics, prostaglandins, antidepressants, anti-psychotic substances, anti-emetics, and imaging agents. The osteoimplant can also possess bone-growth inducing substances that include any of a variety of medically and/or surgically useful substances described below.

Additional components such as described above may be added in any suitable manner. Liquids may inhibit expansion. Accordingly, for components that are added in liquid form, it may be desirable to solidify the liquid in the osteoimplant.

The osteoimplant may be configured for insertion into another implant, such as a vertebral cage. Alternatively, the osteoimplant may be used by itself, for example in a surgical opening or anatomical void, where it is desirable that the osteoimplant expand from a first state to an expanded state. The osteoimplant may absorb blood, and thus may exhibit hemostatic properties, in which case the osteoimplant may be used as a plug.

The osteoimplant may be molded into any suitable shape. Thus, the osteoimplant may be fashioned into a variety of shapes and sizes which are not limited by constraints imposed by, for example, size and/or types of donor bone which are available for construction of the osteoimplant. Example shapes include rectangles, squares, horseshoes, matchsticks, cylinders, beads, pellets, gum-like strips, etc. Those skilled in the art will recognize the applicability of any given shape. For example, shapes such as squares and rectangles are useful in interbody fusion, whether cervical, thoracic, or lumbar, as they efficiently deliver a large volume of implant material and are stackable. Shapes such as matchsticks may be useful for minimally invasive procedures, such as in delivery to an interbody fusion site. Filled cylinders may be used as a seat for a screw or other piece of hardware, or to provide a plug for a burr hole. Rings and cups may be used to deliver cages or other interbody fusion devices. Based on the degree of compression (thus, on the amount of material and the size of the mold) and, for bone, the degree of demineralization, the desired degree of porosity of the osteoimplant may be achieved.

In one embodiment, for use with intervertebral cages, the osteoimplants are provided as 5 mm by 20 mm blocks in three heights: 8 mm, 12 mm, and 16 mm.

Further, the osteoimplant may function as a carrier for, and effectively diffuse, one or more bone-growth inducing substances that promote new bone growth and/or accelerate healing. In such embodiment, the osteoimplant may be used with a carrier device such as an intervertebral fusion device, or it may be used on its own.

CAGES

As shown in Figures 1-3b, the osteoimplant 10 may be configured as an insert that conforms to a graft chamber of a particular device 12 such as a structural spinal device. In one embodiment, the osteoimplant may be used with a graft chamber including a through opening in the superior-inferior direction. When in vivo, the osteoimplant expands within the graft chamber, the expansion being confined by the graft chamber and the vertebral endplates. Thus, to the extent the osteoimplant is able to expand, the osteoimplant makes full contact with the endplates, regardless of whether the endplates are uneven or concave. The osteoimplant thereby increases the potential for successful interbody fusion.

By confining the osteoimplant in one direction, for example with a cage, the osteoimplant is forced to expand in another direction. Thus, the direction of expansion may be controlled as desired for various procedures.

A suitable cage for receiving the osteoimplant is disclosed in PCT Publication No. WO2005/065596 for Intervertebral Implant.

Figure 2 illustrates an intervertebral fusion device 12 for receipt of an osteoimplant 10 in accordance with one embodiment of the present invention. Figure 3a illustrates an osteoimplant 10 inserted into the intervertebral fusion device 12 of Figure 2 in the first state. Figure 3b illustrates an osteoimplant 10 inserted into the intervertebral fusion device 12 of Figure 2 in an expanded state. The depicted intervertebral fusion device 12 is merely an example of one such device that may be used, and is not intended to be limiting. The osteoimplant may be designed and configured to be received into any suitable implant device.

The osteoimplant may be inserted into the intervertebral body fusion device in its first state. As discussed above, the osteoimplant may be configured in shape and size to generally correspond to the interior dimensions of the intervertebral body fusion device. Alternatively, the osteoimplant may be cut and/or manipulated to fit into a device. The osteoimplant may be rehydrated to expand to an increased size via, for example, addition of saline, the patient's own blood, bone marrow aspirate, or water. The amount of expansion may be preset. For example, with particular reference to an embodiment wherein the osteoimplant is molded and dried, the expansion may coincide closely with the dimensions of the expandable material before it was molded to form the insert. Thus, for example, the osteoimplant may be configured to expand as far as permitted by the confines of the intervertebral body fusion device and the spinal endplates, thereby aiding in greater vertebral endplate contact and conformity in spinal surgery.

In one embodiment, the osteoimplant may be used with any suitable device for implantation in the body, such as where that device has an internal chamber. The osteoimplant is thus configured to generally correspond to the dimensions of the internal chamber. Such device may be formed of any suitable material. Example materials include, but are not limited to, metal (such as titanium), polymer, plastic, resorbable material, or bone such as allograft or autograft bone. Further, such device may be any shape and the osteoimplant may be configured via use of a suitable mold for mating with any shape. Thus, for example, the device may be circular, cylindrical, wedge shaped, or shaped as a cage (with or without openings).

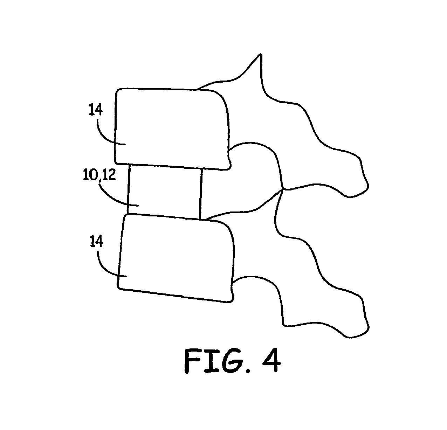

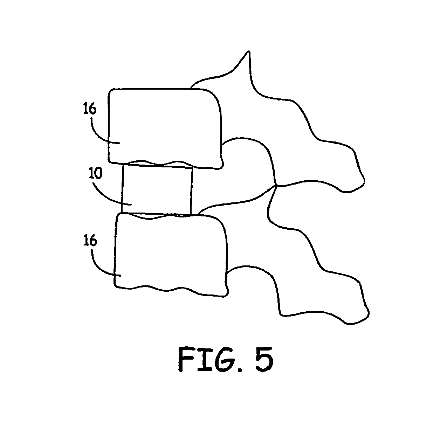

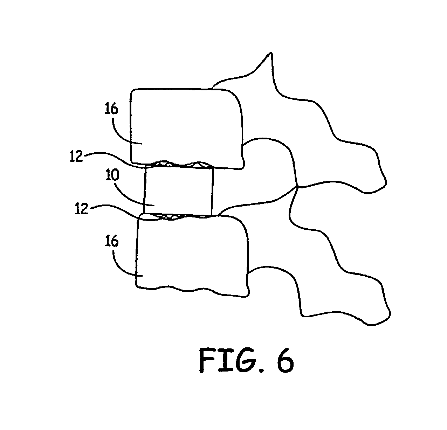

Figures 4-7 illustrate an osteoimplant used with an intervertebral device. Figure 4 illustrates an osteoimplant 10 within an intervertebral device 12 spaced between substantially uniform vertebral endplates 14. Figures 5 and 6 illustrate an osteoimplant 10 within an intervertebral device 12 spaced between non-uniform vertebral endplates 16. As shown in Figure 6, the osteoimplant 10 exhibits osteoconforming^{™} characteristics, expanding to contact the endplates 16. The osteoimplant thereby increases the potential for successful interbody fusion. There are four contributing factors for successful vertebral fusion: osteoinductivity, osteoconductivity, structural support, and maximized endplate contact. As shown, the osteoimplant expands to maximize endplate contact. The intervertebral device provides structural support while the osteoimplant provides osteoinduction and osteoconduction characteristics. Thus, all four of the contributing factors are met by an intervertebral device used with the osteoimplant of the present invention.

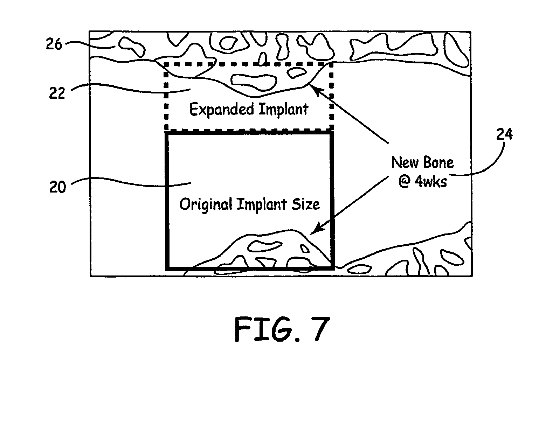

Figure 7 illustrates the size of one embodiment of the implant in its first state 20, the size of the implant as expanded in vivo 22, and the new bone growth 24 at the site at four weeks. As shown, there initially is an approximately 4 mm gap between the implant in its first state 20 and the adjacent vertebral endplate 26. This gap is filled in vivo via expansion of the osteoimplant. Bone growth 24 at the site at approximately four weeks is substantially equal at the top and the bottom of the implant.

**MOLDS**

In certain embodiments, the osteoimplant may be produced using molds. Thus, the configuration of the osteoimplant may be controlled via the configuration of the mold. The shape of the mold may be configured to correspond to a cavity in which the osteoimplant will be fit. For good fit with a cage (or other implant), it may be desirable in some embodiments for the osteoimplant to have smaller overall dimensions than the cage cavity. Thus, for example, the osteoimplant may be approximately 0 to 0.5 mm smaller in length and width than the cage cavity to receive the osteoimplant.

In one embodiment, molds are provided having cavity length and width dimensions approximately equal to those of the corresponding cage. In another embodiment, the molds are prepared having cavity length and width dimensions that are approximately 110% of the corresponding cage cavity dimensions, in which case the cavities are oversized such that, upon shrinkage of most implants during their preparation, they will fall into the range of 0 to 0.5 mm smaller in length and width than the cage cavity. In addition, corners of the molds may have a greater radius than the radius of the internal cavity of the cage to prevent binding that may interfere with implant insertion. In one embodiment, the implant mold height may be approximately 112% of the desired implant height to allow for shrinkage.

**OSTEOIMPLANT COMPRISING BONE PARTICLES**

In certain embodiments, the osteoimplant comprises bone particles. The bone particles may comprise cancellous bone chips, cortical fibers, or a combination thereof. The bone particles may be partially or fully demineralized and/or combined with nondemineralized bone particles and/or other materials. While particular reference is made to bone particles of allograft origin, the bone particles employed in the preparation of the osteoimplant may be obtained from cortical, cancellous, and/or corticocancellous bone that may be of autogenous, allogenic, and/or xenogenic origin. Porcine and bovine bone are types of xenogenic bone tissue that may be used individually or in combination as sources for the bone particles. Particles may be formed by milling whole bone to produce fibers, chipping whole bone, cutting whole bone, fracturing whole bone in liquid nitrogen, or otherwise disintegrating the bone tissue. Particles may optionally be sieved to produce those of a specific size.

In one embodiment, the osteoimplant comprises demineralized cancellous bone particles, which optionally may be supplemented with cortical bone particles. While nondemineralized cancellous bone may function in some load bearing capacity in wet and dry conditions, demineralized cancellous bone acts like a sponge when it is wet and exhibits "memory" properties when dried and subsequently rehydrated. The bone particles may be powdered bone particles possessing a wide range of particle sizes ranging from relatively fine powders to coarse grains and even larger chips. Thus, for example, powdered bone particles may range in average particle size from about 0.05 to about 1.2 cm and preferably from about 0.1 to about 1 cm and possess an average median length to median thickness ratio of from about 1:1 to about 3:1. If desired, powdered bone particles may be graded into different sizes to reduce or eliminate any less desirable size(s) of particles that may be present.

Alternatively, or in combination with the aforementioned bone powder, bone particles generally characterized as elongate and possessing relatively high median length to median thickness ratios may be utilized herein. Such elongate particles are referred to as fibers and may readily be obtained by any one of several methods, e.g., by milling or shaving the surface of an entire bone or relatively large section of bone. Employing a milling technique, one can obtain a mass of elongate bone particles containing at least about 60 weight percent, at least about 70 weight percent, or at least about 80 weight percent of elongate bone particles possessing a median length of from about 2 to about 200 mm or from about 10 to about 100 mm, a median thickness of from about 0.05 to about 2 mm or from about 0.2 to about 1 mm, and a median width of from about 1 mm to about 20 mm or from about 2 to about 5 mm. While any length may be used, longer fibers generally may perform better at holding cancellous bone chips. Longer fibers also may enhance osteoconductivity. The elongate bone particles can possess a median length to median thickness ratio of at least about 50:1 up to about 500:1 or more, or from about 50:1 to about 100:1, and a median length to median width ratio of from about 10:1 and about 200:1, or from about 50:1 to about 100:1. Another procedure for obtaining elongate bone particles, particularly useful for pieces of bone of up to about 100 mm in length, is the bone processing mill described in U.S. Patent No. 5,607,269. Use of this bone mill results in the production of long, thin strips that quickly curl lengthwise to provide tubular-like, coiled bone particles. If desired, elongate bone particles may be graded into different sizes to reduce or eliminate any less desirable size particles that may be present. In overall appearance, elongate bone particles can be described as filaments, fibers, threads, slender or narrow strips, etc. In some uses of the osteoimplant, for example when implanted on its own at a surgical site, the bone particles may be remodeled and partially replaced by new host bone as incorporation of the osteoimplant progresses in vivo.

The osteoimplant may comprise bone particles including demineralized bone particles, nondemineralized bone particles, and combinations thereof. The bone particles can be fully demineralized by removing substantially all of the inorganic mineral content of the bone particles, partially demineralized by removing a significant amount, but less than all, of the inorganic mineral content of the bone particles, or superficially demineralized by removing a minor amount of the inorganic mineral content of the bone particles. Demineralized bone particles induce new bone formation at the site of the demineralized bone and permit adjustment of the overall mechanical properties of the osteoimplant. While the bone particles may be at least partially demineralized, incomplete demineralization of the cancellous and/or of the cortical bone particles provides radiopacity for imaging.

Nondemineralized bone particles possess an initial and ongoing mechanical role, and later a biological role, in the osteoimplant. Superficial or partial demineralization produces particles containing a nondemineralized core. Particles of this type can contribute to the strength of the osteoimplant, through their nondemineralized core. These particles also play a biological role in bringing about new bone ingrowth by the process known as osteoinduction. Full demineralization produces particles in which nearly all of the mineral content has been removed from the particles. Particles treated in this way may contribute to the osteoinductivity of the osteoimplant and provide a coherency or binding effect. The degree of demineralization can be controlled as a function of the duration of treatment (i.e., submersion time in demineralizing agent) and the strength of the treatment medium (i.e., dilute or strong acid). Thus, the degree of "sponginess" or resiliency may be selected to meet a particular clinical application. In one embodiment, the nondemineralized particles contribute to mechanical strength by being of a substantial size. In another embodiment, the nondemineralized bone particles may be bound together in such a manner as to prevent disintegration. In yet another embodiment, the nondemineralized bone particles may be provided in a retaining structure or material. Alternatively, the nondemineralized bone particles may be presented in any suitable manner so as to enhance the osteoimplant's mechanical strength.

The bone particles may be demineralized in accordance with known and conventional procedures in order to reduce their inorganic mineral content. Demineralization methods remove the inorganic mineral component of bone by employing acid solutions. Such methods are well known in the art. See, for example, Reddi et al., Proc. Nat. Acad. Sci. 69, pp. 1601-05 (1972). The strength of the acid solution, the shape of the bone particles, and the duration of the demineralization treatment will determine the extent of demineralization. Reference in this regard may be made to Lewandrowski et al., J Biomed Materials Res, 31, pp 365-372 (1996).

In a suitable demineralization procedure, the bone particles are subjected to a defatting/disinfecting step, followed by an acid demineralization step. In the defatting/disinfecting step, the bone is placed in a solution. An example defatting/disinfectant solution is an aqueous solution of ethanol, the ethanol being a good solvent for lipids and the water being a good hydrophilic carrier to enable the solution to penetrate more deeply into the bone particles. The aqueous ethanol solution also disinfects the bone by killing vegetative microorganisms and viruses. Ordinarily, at least about 10 to about 40 percent by weight of water (i.e., about 60 to about 90 weight percent of defatting agent such as alcohol) should be present in the defatting disinfecting solution to produce optimal lipid removal and disinfection within the shortest period of time. One concentration range of the defatting solution is from about 60 to about 85 weight percent alcohol or about 70 weight percent alcohol. Following defatting, the bone particles are immersed in acid over time to effect their demineralization. Acids that can be employed in this step include inorganic acids, such as hydrochloric acid, and organic acids, such as peracetic acid. After acid treatment, the demineralized bone particles are rinsed with sterile water to remove residual amounts of acid and thereby raise the pH. Where elongate bone particles are employed, some entanglement of the wet demineralized bone particles will result. The wet demineralized bone particles may be immediately shaped into any desired configuration or stored under aseptic conditions, advantageously in a lyophilized state, for processing at a later time. As an alternative to aseptic processing and storage, the particles may be shaped into a desired configuration and sterilized using known methods.

Nondemineralized bone particles may be used in combination with demineralized bone particles to provide additional strength to the osteoimplant. Nondemineralized bone particles act as a stiffener, providing strength to the osteoimplant and enhancing its ability to support load. Nondemineralized bone particles may be used having sufficient size or being bound together to prevent disintegration such that the nondemineralized bone particles may aid the osteoimplant to initially support load. Nondemineralized bone particles also play a biological role in bringing about new bone ingrowth by osteoconduction. Thus, these bone particles are gradually remodeled and replaced by new host bone as incorporation of the osteoimplant progresses over time. Nondemineralized bone particles, however, need not be included in the osteoimplant.

The osteoimplant is formed as follows. A plurality of bone particles are provided. The bone particles may comprise demineralized cancellous bone chips, demineralized cortical fibers, or a combination thereof. Any suitable ratio of bone types may be used. Generally, each of the cancellous bone particles and the cortical bone fibers may be provided as approximately 1 to 100% of the osteoimplant. In one specific embodiment, the osteoimplant may comprise about 50% by weight cancellous bone chips and about 50% by weight cortical bone fibers. The cancellous bone particles may range in size, for example up to approximately 1.2 cm. In one embodiment, the cancellous bone particles and the cortical bone particles each range from approximately 1 mm to approximately 10 mm. Generally, an osteoimplant comprising a substantial majority of cancellous bone chips exhibits good expansion properties but is fragile and may break during handling in the dry state. In contrast, an osteoimplant comprising a substantial majority of cortical bone fibers exhibits reduced expansion properties, but increased osteoinductivity.

Increased compression of the osteoimplant may lead to increased expansion characteristics in the osteoimplant. The osteoimplant may be configured to expand to approximately the precompression size. In one embodiment, compression ranges from approximately 2 mm to approximately 20 mm. Compression may be performed in any suitable manner. In one embodiment, the bone particles are compressed via application of pressure in the direction of desired expansion.

The bone particles (whether cancellous bone chips, cortical bone fibers, or a combination thereof) are compressed, molded together, and, optionally, dried. An osteoimplant substantially free of liquid will exhibit increased expansion characteristics. Thus, it may be desirable, but not necessary, to dry the bone particles. If desired, drying may be accomplished, for example, via lyophilization or freeze-drying. Molding may cause the bone particles to form a solid, compact shape of predetermined dimensions. Generally, the dimensions may correspond to the dimensions of the mold used. The shape may be sized such that it mates with another device, or with an implant or surgical opening. Thus, for example, the mold may be shaped to conform with the interior of a intervertebral body fusion device into which the osteoimplant may be inserted.

After implantation, the osteoimplant may be rehydrated, for example using a physiological saline (water containing 0.9 g NaCl/100 ml water) or water, or the patient's own blood. During rehydration, the freeze-dried solid of the osteoimplant expands to a larger size. Due to the "memory" properties of demineralized cancellous bone, discussed above, the demineralized, cancellous bone particles may be molded into any suitable shape to fill correspondingly sized or shaped cavities, or in geometries that are used to expand and fill any given shape smaller than or equal to the expanded size of the osteoimplant. In addition, the degree of expansion from compression (i.e., as a function of the volume of void to be filled) may be used to produce an implant, such as a demineralized cancellous body, with particular porosity. Swelling agents other than or in addition to physiological saline and/or water also may be used.

**OSTEOIMPLANT COMPRISING SYNTHETIC MATERIAL**

In certain embodiments, the osteoimplant comprises a synthetic material, i.e., a material other than bone. The synthetic material can be any material suitable for use in the present invention. In one embodiment, the synthetic material may be an expandable polymer. In another embodiment, the material may be a compressed, dried hydrogel. Further, the osteoimplant may comprise a pseudo-synthetic material such as a collagen or cellulose sponge, which may be cross-linked or otherwise processed. The osteoimplant may also comprise combinations of these materials, and may further comprise bone. Any suitable material may be used but should generally be expandable, biocompatible, and lack immunogenicity.

In another embodiment, the osteoimplant may comprise a chemically-cross-linked glycosaminoglycan (GAG) hydrogel such as taught by Kirker, et. al. "Glycosaminoglycan Hydrogel Films as Bio-Interactive Dressings for Wound Healing"; Biomaterials 23 (2002) 3661-71. Such hydrogel may comprise hyaluronan, hyaluronic acid, chondroitin sulfate (CS), or glycosaminoglycan (GAG) Hyaluronic acid and hyaluronan are non-immunogenic and form highly viscous aqueous solutions. GAG molecules may be chemically modified to tailor their physiochemical and mechanical properties while retaining their natural bio-compatibility, bio-degradability, and lack of immunogenicity.

In still other embodiments, the osteoimplant may comprise any material, such as a polymer, that is expandable. Examples of other suitable polymers include fluoropolymers, polyolefins, and other suitable polymers. Suitable fluoropolymers include homopolymers of polytetrafluoroethylene and copolymers of polytetrafluoroethylene in which the co-monomer is ethylene, chlorotrifluoroethylene, perfluoroalkoxytetrafluoroethylene, and fluorinated propylene. Suitable Polyolefins include polypropylene and polyethylene. In addition, polyethylene terephthalate, polystyrene, and ultra high molecular weight polyethylene also may be suitable expandable materials.

In another embodiment, the osteoimplant may comprise an elastic hydrogel such as described in L. Sefc et. al. "Development of Hydrogel Implants for Urinary Incontinence Treatment"; Biomaterials 23 (2002) 3711-3715.

In addition to expansion properties, the material may exhibit osteoinductive and/or osteoconductive properties. For example, synthetic materials and cancellous bone may exhibit osteoconductive properties, while demineralized cortical bone particles may exhibit osteoinductive properties.

Furthermore, any of the osteoimplants described above, whether bone, synthetic material, or a combination thereof, may be treated to enhance osteoinductivity, as described in Bone Matrix Compositions and Methods, K. Benham et al., PCT Patent Publication No. WO2007/053850.

**Example 1**

### Formation of osteoimplants:

### 1:1 ratio cancellous chips:cortical fibers

For a 7mm implant, inserts were compressed to 4mm.
For a 15 mm implant, inserts were compressed to 7 mm.
Inserts were molded using SLA molds.
Molds were placed in -70 freezer for one hour, and then freeze dried using a freeze mobile for 24 hours.
Implants numbered piece 11-piece 16. Pieces 11, 12, and 13 were for a 7 mm implant. Pieces 14, 15, and 16 were for a 15 mm implant.

### Testing:

After freeze drying, all inserts were weighed and measured using a calibrated digital caliper.

Measurements were taken at 5 minutes, 60 minutes, and 24 hours while rehydrating. All measurements are in mm.

Results

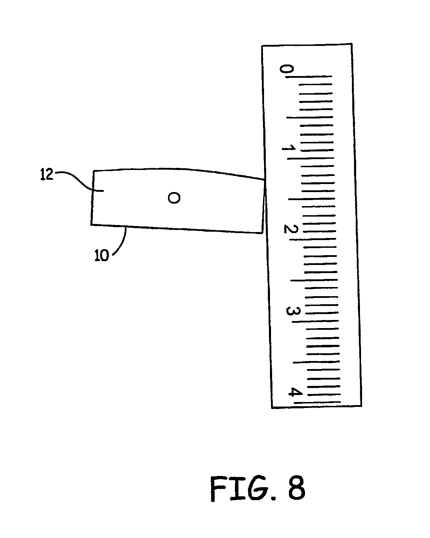

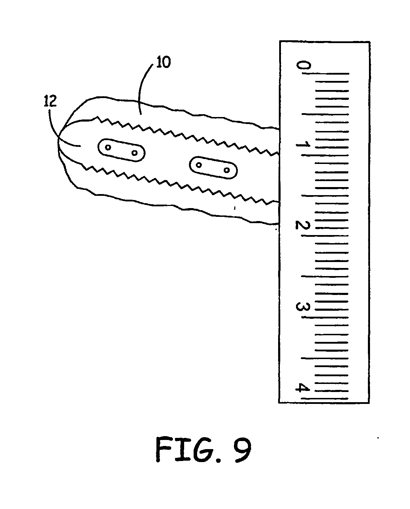

Figure 8 illustrates an osteoimplant after 0 minutes rehydration. Figure 9 illustrates an osteoimplant after 30 minutes rehydration.

### Piece 11

| | Chip Count | Weight | Length | Width | Height | Expansion |
|---|---|---|---|---|---|---|
| Post lyophilization (0 min.) | | 0.31 | 17.28 | 5.39 | 6.3 | |
| 5 min. rehydration | | | | | 10.0 | 3.7 |
| 60 min rehydration | | | | | 12.21 | 5.91 |
| 24 hour rehydration | | | | | 14.03 | 7.73 |
| Chip Count | 5 | | | | | |

### Piece 12

| | Chip Count | Weight | Length | Width | Height | Expansion |
|---|---|---|---|---|---|---|
| Post lyophilization (0 min.) | | 0.33 | 17.58 | 5.22 | 5.93 | |
| 5 min. rehydration | | | | | 9.64 | 3.71 |
| 60 min rehydration | | | | | 15.48 | 9.55 |
| 24 hour rehydration | | | | | 17.11 | 11.18 |
| Chip count | 8 | | | | | |

### Piece 13

| - | Chip Count | Weight | Length . | Width | Height | Expansion |
|---|---|---|---|---|---|---|
| Post lyophilization (0 min.) | | 0.37 | 17.86 | 5.26 | 6.16 | |
| 5 min. rehydration | | | | | 9.1 | 2.94 |
| 60 min rehydration | | | | | 18.8 | 12.64 |
| 24 hour rehydration | | | | | 21.05 | 14.89 |
| Chip count | 10 | | | | | |

### Piece 14

| | Chip Count | Weight | Length | Width | Height | Expansion |
|---|---|---|---|---|---|---|
| Post lyophilization (0 min.) | | 0.47 | 17.46 | 5.32 | 13.1 | |
| 5 min. rehydration | | | | | 17.01 | 3.91 |
| 60 min rehydration | | | | | 21.56 | 8.46 |
| 24 hour rehydration | | | | | 24.56 | 11.46 |
| Chip count | 10 | | | | | |

### Piece 15

| | Chip Count | Weight | Length | Width | Height | Expansion |
|---|---|---|---|---|---|---|
| Post lyophilization (0 min.) | | 0.51 | 17.85 | 5.34 | 12.9 | |
| 5 min. rehydration | | | | | 16.53 | 3.63 |
| 60 min rehydration | | | | | 21.54 | 8.64 |
| 24 hour rehydration | | | | | 21.58 | 8.68 |
| Chip count | 8 | | | | | |

### Piece 16

| | Chip Count | Weight | Length | Width | Height | Expansion |
|---|---|---|---|---|---|---|
| Post lyophilization (0 min.) | | 0.48 | 18.03 | 5.14 | 13.13 | |
| 5 min. rehydration | | | | | 17.81 | 4.68 |
| 60 min rehydration | | | | | 19.81 | 6.68 |
| 24 hour rehydration | | | | | 20.23 | 7.1 |
| Chip count | 10 | | | | | |

### Results for 7 mm implant

| | Chip | Weight | Expansion |
|---|---|---|---|
| | 5 | 0.31 | 7.73 |
| | 8 | 0.33 | 11.18 |
| | 10 | 0.37 | 14.89 |
| Average | 7.7 | 0.34 | 11.27 |
| Standard Deviation | 2.5 | 0.03 | 3.58 |

### Results for 15 mm implant

| | Chip | Weight | Expansion |
|---|---|---|---|
| | 10 | 0.47 | 11.46 |
| | 8 | 0.51 | 8.68 |
| | 10 | 0.48 | 7.1 |
| Average | 9.3 | 0.49 | 9.08 |
| Standard Deviation | 1.2 | 0.02 | 2.21 |

Bone insert functional fit experiments were conducted to test for fit of the bone insert into various intervertebral fusion devices. The experiments looked at rehydrated bone and nonrehydrated bone.

An attempt was made to insert the osteoimplant into an opening of an intervertebral fusion device. In some cases, the bone was rehydrated for one minute in saline in a beaker to facilitate an easier fit. In others, manipulation, such as cutting the osteoimplant, was used to fit the osteoimplant. Observations were made relating to damage and whether the osteoimplant could be fully inserted. The device and insert were placed in saline in a beaker and allowed to continue rehydrating. Observations of expansion over time demonstrated that, regardless of the means used to fit the osteoimplant into the fusion device, the osteoimplant maintained its expansion characteristics.

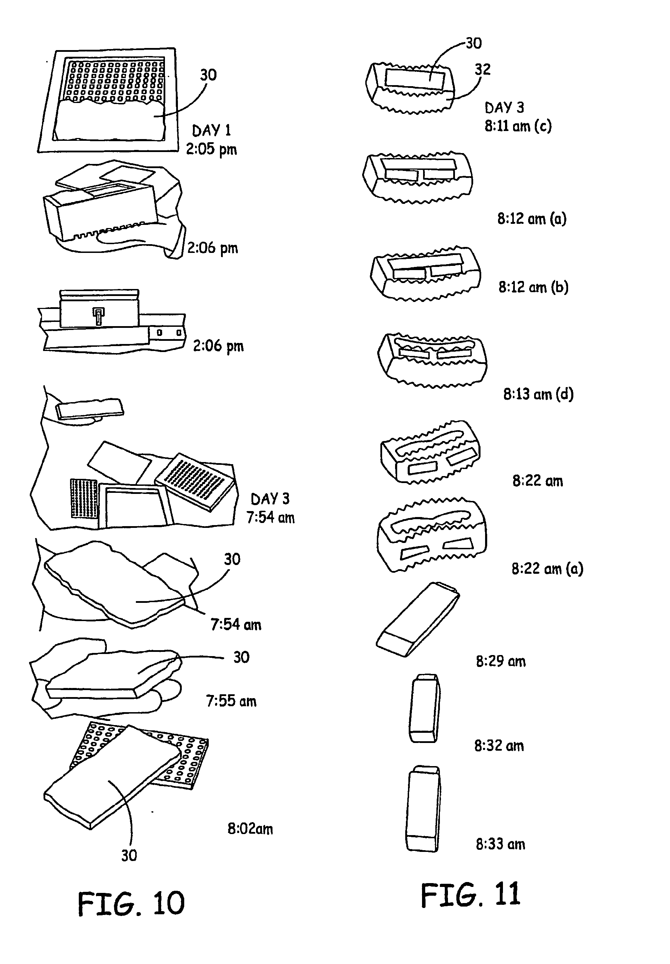

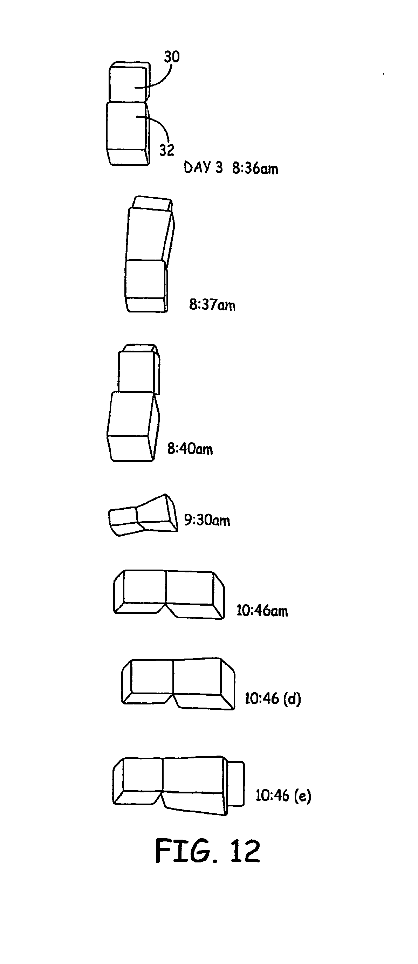

Figures 10-12 illustrate Insertion/Expansion testing of an osteoimplant 30 in accordance with one embodiment of the present invention. As shown, the osteoimplant 30 is formed in Figure 10 and tested within a device 32 in Figures 11 and 12.

**Example 2**

Table I illustrates the results of studies evaluating the expansion characteristics of osteoimplants comprising 100% fibers versus osteoimplants comprising 100% chips. In the osteoimplants comprising 100% cancellous bone chips, the chips ranges in size from 1.7 mm to 10 mm. The test implants were made using 10 mm compression.

| 100% Fibers | | | | | 100% Chips | | | | | |
|---|---|---|---|---|---|---|---|---|---|---|
| | | | | | | | | | | |
| | Start | 60 min. | Expansion | % Expansion | | Start | 60 min. | Expansion | % Expansion | Chip Count |
| | 14.53 | 16.61 | 2.08 | 14.3 | | 14.08 | | | | 15 |
| | 14.25 | 16.94 | 2.69 | 18.9 | | 13.78 | 17.93 | 4.15 | 31.0 | 17 |
| | 14.86 | 16.89 | 2.03 | 13.7 | | 13.85 | 19.31 | 5.46 | 39.4 | 17 |

**Table I**

| | | | | | | | | | | |
|---|---|---|---|---|---|---|---|---|---|---|
| | 14.55 | 16.81 | 2.27 | 15.6 | | 13.90 | 18.62 | 4.81 | 34.6 | 16.33 |
| Avg Dev | 0.31 | 0.18 | 0.37 | | Avg Dev | 0.16 | 0.98 | 0.93 | | 1.15 |
| | | | | | | | | | | |

| | Start | 60 min. | Expansion | % Expansion | | Start | 60 min. | Expansion | % Expansion | Chip Count |
|---|---|---|---|---|---|---|---|---|---|---|
| | 6.99 | 8.39 | 1.4 | 20.0 | | 6.99 | 15.49 | 8.50 | 121.6 | 13 |
| | 6.75 | 8.60 | 1.85 | 27.4 | | 6.77 | 15.00 | 8.23 | 121.6 | 12 |
| | 6.93 | 8.61 | 1.68 | 24.2 | | 6.82 | 15.39 | 8.57 | 125.7 | 13 |
| | 6.89 | 8.53 | 1.64 | 23.8 | | 6.86 | 15.29 | 8.43 | 122.9 | 12.67 |
| Avg Dev | 0.12 | 0.12 | 0.23 | | Avg Dev | 0.12 | 0.26 | 0.18 | | 0.58 |

As may be seen from Table I, the osteoimplants comprising 100% fibers generally expanded about 2 mm. The osteoimplants comprising 100% chips generally expanded about 14 mm. Thus, in these embodiments, the fibers expanded less than their compression while the chips expanded more than their compression.

**Example 3**

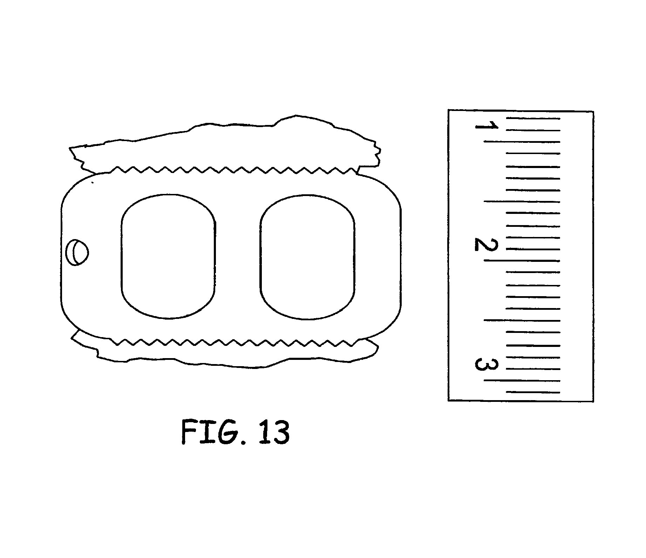

Expansion Measurement Techniques and Expansion Data. When used within another implant (such as a vertebral fusion cage), the osteoimplant is confined on its sides by the cage and on its ends by the vertebral end plates. Figure 13 illustrates sample expansion of an osteoimplant 40 in a vertebral cage 42. As shown, the chips and fibers tend not to extrude out of the cage windows. Equivalent expansion of the implants while they were inserted in cages was measured. The dry dimensions were measured before the implants were put in the cage. During hydration, as the implants expanded, material extended beyond the tops of the cages. Calipers were used to measure the total height of the implants, as they extended beyond both ends of the cages. The difference between the hydrated height and the dry height was the expansion.

For most runs, the expansion as a function of time was determined by taking measurements at 10 or 15 minute intervals up to one hour. Sometimes additional measurements beyond an hour were recorded. Expansion was generally complete after one hour, and only one hour time points were used to report expansion numbers.

As discussed above, expansion at 60 minutes was used as the basis for comparing one sample to another. The data was analyzed to see what effect process variables such as compression amount, chip count, implant weight, and implant size had on expansion. Although expansion values varied, most samples met the goal of 4 mm expansion.

While there were differences in how expansion was measured, for each run the data were gathered using consistent methods. The run and sample identification is summarized in Table II below.

**Table II**

| Run ID | Sequence number | Mold type | Compression | Exp Mean; std. dev. |
|---|---|---|---|---|
| Run 2 | 11-13 | Lumbar, small | 4 | 9.4; 3.4 |
| | 14-16 | Lumbar, large | 8 | 7.9; 1.1 |
| Run 3 | 1-3 | Lumbar, small | 4 | 5.4; 0.5 |
| | 4-6 | Lumbar, large | 8 | 5.4; 0.3 |
| Run 3a | 7-9 | Lumbar, small | 15 | 14.3; 2.5 |
| | 10-12 | Lumbar, large | 15 | 14.5; 3.5 |
| Run 4 | 1-3 | Lumbar, small | 8 | 4.5; 1.2 |
| | 4-6 | Lumbar, large | 8 | 4.4; 1.1 |
| Run 4a | 7-9 | Lumbar, small | 8 | 2.2; 0.5 |
| | 10-12 | Lumbar, large | 8 | 3.3; 0.5 |
| Run 4b | 13-15 | Lumbar, small | 8 | 2.3; 0.05 |
| | 16-18 | Lumbar, large | 8 | 3.1; 0.2 |
| Run 7 | 1-3 | Lumbar, small | 10 | 6.7; 1.3 |
| | 4-6 | Lumbar, large | 10 | 4.7; 0.2 |
| | 7-9 | Lumbar, small | 10 | 4.8; 0.3 |
| | 10-12 | Lumbar, large | 10 | 6.1; 0.6 |
| | 13-15 | Lumbar, small | 10 | 7.6; 1.2 |
| | 16-18 | Lumbar, large | 10 | 7.4; 1.5 |
| | 19-21 | Lumbar, small | 10 | 6.5; 0.1 |
| | 22-24 | Lumbar, large | 10 | 5.6; 0.6 |
| Run 8 | 1-12 | Lumbar, large | 10 | 4.8; 0.8 |
| | 13-24 | Lumbar, small | 10 | 3.7; 0.6 |
| Run 9 | 1-12 | Lumbar, large | 10 | 4.5; 0.4 |

Table III lists compression and expansion for various implant types:

| Implant Type | Compression | Run # | Expansion, mm |
|---|---|---|---|
| Tall lumbar | 10 mm | 9 | 4.5 +/- 0.4 |
| | | | |
| Tall lumbar | 10 mm | 8 | 4.8 +/- 0.8 |
| Short lumbar | 10 mm | 8 | 3.7 +/- 0.6 |

**Table III**

| | | | |
|---|---|---|---|
| Tall lumbar | 10 mm | 7 | 6.0 +/- 1.0 |
| Short lumbar | 10 mm | 7 | 6.1 +/- 0.9 |
| | | | |
| Tall lumbar | 8 mm | 4 | 4.8 +/- 1.6 |
| Short lumbar | 8 mm | 4 | 3.0 +/- 0.9 |

Table IV lists expansion values for various example embodiments.

**Table IV**

| **Expansion Values** | | | | |
|---|---|---|---|---|
| 4 mm compressed samples | 15 mm compressed samples | | 8 mm compressed samples | 10 mm compressed samples |
| 7.4 +/- 0.7 mm | 14.4 mm +/- 0.7 mm | | 4.1 mm +/- 0.4 mm | 5.0 mm +/- 0.9 mm |

**Example 4**

A glycosaminoglycan hydrogel is prepared as described in: Kirker, et. al. "Glycosaminoglycan Hydrogel Films as Bio-Interactive Dressings for Wound Healing"; Biomaterials 23 (2002) 3661-3671. As described in Kirker, either of hyaluronan (HA) or chondroitin sulfate (CS) glycosaminoglycan (GSG) may be used.

HA-ADH is synthesized: HA is dissolved in water at 5 mg/ml. 5 M equivalent of adipic dihydrazide (ADH) and 3 equivalent of 1-Ethyl-3-[3-(dimethylamino)-propyl]carbodiimide (EDCI) are added in solid form, while maintaining the pH at 4.75 by the addition of 1.0 N HCL. The reaction is stopped by raising the pH of reaction mixture to 7.0, followed by exhaustive dialysis. The solution is then centrifuged, and the supernatant lyophilized.

CS-ADH is synthesized: Chondroitin sulfate (CS) is dissolved in water at 25 mg/ml. 5 M equivalent of adipic dihydrazide (ADH) and 2 equivalent of 1-Ethyl-3-[3-(dimethylamino)-propyl]carbodiimide (EDCI) are added in solid form, while maintaining the pH at 4.75 by the addition of 1.0 N HCL. The reaction is stopped by raising the pH of reaction mixture to 7.0, followed by exhaustive dialysis. The solution is then centrifuged, and the supernatant lyophilized.

The GAG-ADH (either HA-ADH or CS-ADH) is dissolved in water (5 mg/ml for HA-ADH and 25 mg/ml for CS-ADH) to form Solution A. PEG-diald is dissolved in water at a concentration of 50 mg/ml to form Solution B. Volumes of Solutions A and B are added to a small polystyrene dish to give desired equivalents of aldehyde and hydrazide functionalities, and the solutions are mixed with gentle swirling. A hydrogel forms within 60 seconds at room temperature. The mixture is agitated gently on an orbital platform for an additional 24 hours at 25 degrees Celsius to obtain a solid, uniform hydrogel. The hydrogel is stored overnight to allow solvent evaporation and thus provide a flexible HA film, which swells when rehydrated in aqueous solutions. The HA-ADH films may be prepared using an aldehyde:ADH ratio of 1:1. The CS-ADH films may be prepared with a ratio of 1:4.

The hydrogel is solvent cast with water into cylindrical 90 mm diameter beakers to a depth of about 30 mm. After casting, the material is put in a 40 degree C oven until dry. Upon removal from the oven, the cast sheets are removed from the beakers and chopped into particles about 3 to 6 mm in size. The hydrogel particles are mixed with wet DBM fibers in a 50/50 volume ratio and put into implant molds. The molds are put in a 40 degree C oven until dry and then removed and packaged. Upon hydration (after implantation), the hydrogel particles expand and cause the entire implant to expand.

**Example 5**

A glycosaminoglycan hydrogel is prepared as described in Kirker, et. al. "Glycosaminoglycan Hydrogel Films as Bio-Interactive Dressings for Wound Healing"; Biomaterials 23 (2002) 3661-3671 and discussed in Example 3, above.

The hydrogel solution is mixed with DBM fibers (25/75 DBM/Hydrogel solution) to form a fiber-hydrogel slurry. The fiber-hydrogel slurry is poured into implant molds then put in a 40 degree C oven until dry. The molds are oversized such that the dry product is of the desired size. After implantation, the hydrogel portion swells and causes the implant to expand to fill the defect space.

**Example 6**

A 2-hydroxyethyl methacrylate hydrogel supplemented with methacrylate acid is prepared as described in L. Sefc et. al. "Development of Hydrogel Implants for Urinary Incontinence Treatment"; Biomaterials 23 (2002) 3711-15. 2-Hydroxyethyl methacrylate (HEMA) is mixed with freshly distilled methacrylic acid. A solution of ammonium persulfate in water is added. This solution is injected into a silicone tube with a syringe and the second end of the tube is closed. The tube is immersed into a 5% solution of sodium pyrosulphite for 24 hours. Thereafter, both ends of the tube are opened and inserted into hexane (10 min), polymer being taken out and washed by ethanol (24 hours), water (3 days), 1% solution of sodium hydroxide (1 day), and finally by water again (10 days, fresh water changed daily). The hydrogel is cut to 15 mm rolls that are freely dried in open air (2-3 days) and placed into a vial and closed. This method is carried out in a pure sterile room (max 100 particles in 1 m³) under a germicide lamp.

The resulting particles are 1.6 mm in diameter with a length of 4.5 mm. The hydrogel particles are mixed with wet DBM fibers in a 50/50 volume ratio and put into implant molds. The molds are put in a 40 degree C oven until dry and then removed and packaged. Upon hydration (after implantation), the hydrogel particles expand to 3.9 mm in diameter and 11 mm in length, causing the entire implant to expand.

**Example 7**

A cellulose sponge is rehydrated and cut into 4 mm cubes. The 4 mm cubes are mixed with wet DBM fibers in a 50/50 volume ration and put into implant molds. The molds are placed in a -70 degree C freezer until frozen, then placed in a lyophilizer until dried

## Claims

1. An osteoimplant (10) comprising:
demineralized cancellous bone particles; and
demineralized cortical bone particles;
**characterized in that** the osteoimplant exhibits a first state (20) and an expanded state (22), the expanded state being achieved upon implantation of the osteoimplant.

2. The osteoimplant (10) of claim 1, wherein the demineralized cancellous bone particles are demineralized cancellous bone chips in the size range of 1 to 10 mm.

3. The osteoimplant (10) of claim 1, wherein the demineralized cortical bone particles are demineralized cortical bone fibers in the size range of 1 to 10 mm.

4. The osteoimplant (10) of claim 1, wherein the osteoimplant is at least 4 mm larger in one direction in the expanded state (22) than in the first state (20).

5. The osteoimplant (10) of claim 1, wherein the osteoimplant (10) is conformable with an interior of a device (12) such that the osteoimplant (10) may be inserted into the device (12).

6. The osteoimplant (10) of claim 1, further comprising a biocompatible additive.

7. The osteoimplant (10) of claim 1, wherein the osteoimplant (10) is at least approximately ten percent larger in one direction in the expanded state (22) than in the first state (20).

8. The osteoimplant (10) of claim 1, wherein the implant is osteoinductive.

9. The osteoimplant (10) of claim 1. wherein the implant is osteoconductive.

10. The osteoimplant (10) of claim 1, wherein the implant is constrained by a biocompatible, biodegradable membrane.

11. The osteoimplant (10) of claim 10, wherein the membrane disintegrates upon exposure to moisture.

12. The osteoimplant (10) of claim 1, wherein the implant is dried in the first state (20).

13. The osteoimplant (10) of claim 1, wherein the osteoimplant (10) is load-bearing.

14. The osteoimplant (10) of claim 1, wherein the osteoimplant (10) is blood absorbent and acts as a hemostatic plug.

15. The osteoimplant (10) of claim 1, wherein the expanded state (22) varies from the first state (20) in only one direction.

16. The osteoimplant (10) of according to any one of claims 1 - 15, the expanded state (22) being achieved upon exposure to liquid *in vivo.*

17. An implant system comprising an osteoimplant (10) according to any one of claims 1-16 and
an intervertebral fusion device (12); the implant (10) being positioned within the intervertebral fusion device (12);
wherein the implant (10) extends beyond the intervertebral fusion device (12) in at least one direction when in the expanded state (22).

18. The implant system of claim 17, wherein the intervertebral fusion device (12) controls a direction of expansion of the implant (10).

19. The implant system of claim 17, wherein the implant system is conformable with geometry of adjacent vertebral bodies after being implanted.

## Patentansprüche

1. Osteoimplantat (10), das Folgendes umfasst:
demineralisierte Spongiosa-Partikel; und
demineralisierte kortikale Knochenpartikel;
**dadurch gekennzeichnet, dass** das Osteoimplantat einen ersten Zustand (20) und
einen ausgedehnten Zustand (22) zeigt, wobei der ausgedehnte Zustand nach Implantation des Osteoimplantats erreicht wird.

2. Osteoimplantat (10) nach Anspruch 1, worin die demineralisierten Spongiosa-Partikel demineralisierte Spongiosa-Späne im Größenbereich von 1 bis 10 mm sind.

3. Osteoimplantat (10) nach Anspruch 1, worin die demineralisierten kortikalen Knochenpartikel demineralisierte kortikale Knochenfasern im Größenbereich von 1 bis 10 mm sind.

4. Osteoimplantat (10) nach Anspruch 1, worin das Osteoimplantat im ausgedehnten Zustand (22) in einer Richtung mindestens 4 mm größer ist als im ersten Zustand (20).

5. Osteoimplantat (10) nach Anspruch 1, worin das Osteoimplantat (10) mit einem Inneren einer Vorrichtung (12) übereinstimmt, so dass das Osteoimplantat (10) in die Vorrichtung (12) eingesetzt werden kann.

6. Osteoimplantat (10) nach Anspruch 1, das weiter ein biologisch verträgliches Additiv umfasst.

7. Osteoimplantat (10) nach Anspruch 1, worin das Osteoimplantat (10) im ausgedehnten Zustand (22) in einer Richtung mindestens ungefähr zehn Prozent länger ist als im ersten Zustand (20).

8. Osteoimplantat (10) nach Anspruch 1, worin das Implantat osteoinduktiv ist.

9. Osteoimplantat (10) nach Anspruch 1, worin das Implantat osteokonduktiv ist.

10. Osteoimplantat (10) nach Anspruch 1, worin das Implantat durch eine biologisch verträgliche, biologisch abbaubare Membran eingeschränkt ist.

11. Osteoimplantat (10) nach Anspruch 10, worin sich die Membran bei Aussetzung gegenüber Feuchtigkeit zersetzt.

12. Osteoimplantat (10) nach Anspruch 1, worin das Implantat im ersten Zustand (20) getrocknet ist.

13. Osteoimplantat (10) nach Anspruch 1, worin das Osteoimplantat (10) lasttragend ist.

14. Osteoimplantat (10) nach Anspruch 1, worin das Osteoimplantat (10) blutabsorbierend ist und als hämostatischer Stopfen agiert.

15. Osteoimplantat (10) nach Anspruch 1, worin sich der ausgedehnte Zustand (22) vom ersten Zustand (20) in nur einer Richtung unterscheidet.

16. Osteoimplantat (10) nach einem der Ansprüche 1-15, wobei der ausgedehnte Zustand (22) bei Aussetzung gegenüber Flüssigkeit *in vivo* erreicht wird.

17. Implantatsystem, das ein Osteoimplantat (10) nach einem der Ansprüche 1-16 und eine intervertebrale Fusionsvorrichtung (12) umfasst; wobei das Implantat (10) innerhalb der intervertebralen Fusionsvorrichtung (12) positioniert ist;
worin sich das Implantat (10) in mindestens einer Richtung über die intervertebrale Fusionsvorrichtung (12) hinaus erstreckt, wenn es sich im ausgedehnten Zustand (22) befindet.

18. Implantatsystem nach Anspruch 17, worin die intervertebrale Fusionsvorrichtung (12) eine Ausdehnungsrichtung des Implantats (10) kontrolliert.

19. Implantatsystem nach Anspruch 17, worin das Implantatsystem, nachdem es implantiert ist, mit der Geometrie der benachbarten Wirbelkörper übereinstimmt.

## Revendications

1. Implant osseux (10) comprenant :
des particules d'os spongieux déminéralisé ; et
des particules d'os cortical déminéralisé ;
**caractérisé en ce que** l'implant osseux présente un premier état (20) et un état expansé (22), l'état expansé étant obtenu lors de l'implantation de l'implant osseux.

2. Implant osseux (10) de la revendication 1, dans laquelle les particules d'os spongieux déminéralisé sont des fragment d'os spongieux déminéralisé d'une taille allant de 1 à 10 mm.

3. Implant osseux (10) de la revendication 1, dans laquelle les particules d'os cortical déminéralisé sont des fibres d'os cortical déminéralisé d'une taille allant de 1 à 10 mm.

4. Implant osseux (10) de la revendication 1, dans laquelle l'implant osseux est au moins 4 mm plus large dans une direction à l'état expansé (22) que dans le premier état (20).

5. Implant osseux (10) de la revendication 1, dans laquelle l'implant osseux (10) est conformable à l'intérieur d'un dispositif (12) de sorte que l'implant osseux (10) peut être inséré dans le dispositif (12).

6. Implant osseux (10) de la revendication 1, qui comprend également un additif biocompatible.

7. Implant osseux (10) de la revendication 1, dans laquelle l'implant osseux (10) est au moins dix pour cent plus large environ dans une direction à l'état expansé (22) que dans le premier état (20).

8. Implant osseux (10) de la revendication 1, dans laquelle l'implant est ostéoinducteur.

9. Implant osseux (10) de la revendication 1, dans laquelle l'implant est ostéoconducteur.

10. Implant osseux (10) de la revendication 1, dans laquelle l'implant est renfermé dans une membrane biocompatible biodégradable.

11. Implant osseux (10) de la revendication 10, dans laquelle la membrane se désintègre à l'exposition à l'humidité.

12. Implant osseux (10) de la revendication 1, dans laquelle l'implant est séché dans le premier état (20).

13. Implant osseux (10) de la revendication 1, dans laquelle l'implant osseux (10) est un implant porteur.

14. Implant osseux (10) de la revendication 1, dans laquelle l'implant osseux (10) permet une absorption du sang et fait office de clou hémostatique.

15. Implant osseux (10) de la revendication 1, dans laquelle l'état expansé (22) varie par rapport au premier état (20) dans une seule direction.

16. Implant osseux (10) selon l'une quelconque des revendications 1-15, l'état expansé (22) étant produit lors d'une exposition à un liquide *in vivo.*

17. Système d'implant comprenant un implant osseux (10) selon l'une quelconque des revendications 1-16 et
un dispositif de fusion intervertébrale (12), l'implant (10) étant positionné à l'intérieur du dispositif de fusion intervertébrale (12) ;
l'implant (10) se prolongeant en-delà du dispositif de fusion intervertébrale (12) dans au moins une direction quand il est à l'état expansé (22).

18. Système d'implant de la revendication 17, dans laquelle le dispositif de fusion intervertébrale (12) contrôle l'expansion de l'implant (10) dans une direction.

19. Système d'implant de la revendication 17, dans laquelle le système d'implant est adaptable à la géométrie de corps vertébraux adjacents une fois implanté.
